## Europäisches Patentamt

## European Patent Office

## Office européen des brevets

(11) Veröffentlichungsnummer: **0 264 805**
**A2**

## (12) EUROPÄISCHE PATENTANMELDUNG

(21) Anmeldenummer: **87114995.1**

(22) Anmeldetag: **14.10.87**

(51) Int. Cl.4: **C12N 15/00 , C12N 9/10**

Der Anmelder hat eine Erklärung nach Regel 28 (4) EPÜ (Herausgabe einer Probe nur an einen Sachverständigen) eingereicht.
Eingangsnummer(n) der Hinterlegung(en): DSM 2397.

(30) Priorität: **20.10.86 DE 3635583**

(43) Veröffentlichungstag der Anmeldung:
**27.04.88 Patentblatt 88/17**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Marquardt, Rüdiger, Dr.**
**Günthersburgallee 69**
**D-6000 Frankfurt am Main(DE)**
Erfinder: **Präve, Paul, Prof. Dr.**
**Kronberger Weg 7**
**D-6231 Sulzbach Taunus(DE)**
Erfinder: **Esser, Karl, Prof. Dr.**
**Am Spik 23a**
**D-4630 Bochum(DE)**
Erfinder: **Stahl, Ulf, Prof. Dr.**
**Mühlenfeldstrasse 115**
**D-1000 Berlin(DE)**
Erfinder: **Winnacker, Ernst-Ludwig, Prof. Dr.**
**Dall-Armistrasse 41a**
**D-8000 München(DE)**

(54) **Hybridplasmid zur Replikation in methylotrophen Mikroorganismen und Verfahren zu dessen Herstellung.**

(57) Aus dem Plasmid pBE 3 aus Methylomonas clara DSM 2397 wird mit Hilfe des Restriktionsenzyms Not I ein Bereich kartiert und isoliert, der dem Replikationsursprung entspricht. Durch Verbindung dieses Replikationsursprungs mit beliebigen anderen Plasmiden können Hybride hergestellt werden, die in methylotrophen Bakterien stabil zu replizieren vermögen.

FIG.1

## Hybridplasmid zur Replikation in methylotrophen Mikroorganismen und Verfahren zu dessen Herstellung

In der Europäischen Patentanmeldung 0 098 967 wird ein Verfahren zur Herstellung von obligat methylotrophen, Fremd-DNA exprimierenden Bakterien beschrieben sowie dafür geeignete Plasmide und Wirtsorganismen. Dazu wird ein Plasmid aus obligat methylotrophen Bakterien mit einem E. coli Vektor zu einem Hybridplasmid verknüpft, das wiederum durch Konjugation in methylotrophe Bakterien transferiert werden kann.

Eines der in Frage kommenden Plasmide aus den obligat methylotrophen Bakterien, das pBE 3 aus Methylomonas clara, DSM 2397, ist jedoch mit einem Molgewicht von 10 MD verhältnismäßig groß. Aus Gründen der bequemeren Handhabung ist die Zerlegung dieses Plasmids erwünscht. Dies hat sich jedoch als schwierig herausgestellt, da das Plasmid für die meisten Enzyme mit hexamerer Erkennungssequenz keine oder nur wenige Schnittstellen aufweist (EP-A 0 098 967).

Überraschenderweise läßt sich das Plasmid pBE 3 jedoch mit dem Restriktionsenzym Not I, welches eine oktamere Sequenz erkennt, in mindestens 9 Fragmente aufspalten. Es konnte gezeigt werden, daß die für eine Replikation in M. clara notwendige und ausreichende Information auf dem größten dieser Not I Fragmente lokalisiert ist. Dieses Fragment ist entweder in Vektoren einzubauen, die ebenfalls Not I Schnittstellen besitzen, oder kann so verändert werden, daß es durch Verdauung mit anderen Enzymen aus den jeweiligen Hybridplasmiden zugänglich wird und in Vektoren mit den entsprechenden Schnittstellen eingebaut werden kann.

Die Erfindung betrifft somit:

1. Hybridplasmide mit einem Fragment aus pBE 3, das für die Replikation in Methylomonas clara verantwortlich ist.

2. Ein Verfahren zur Herstellung der unter 1) genannten Hybridplasmide, das dadurch gekennzeichnet ist, daß man das Plasmid-Fragment von pBE 3 mit einem Plasmid mit Selektionsmarkern ligiert.

3. Die Verwendung der unter 1) genannten Hybridplasmide zur Konstruktion von Vektoren für methylotrophe Bakterien.

Die Erfindung wird im folgenden, insbesondere in ihren bevorzugten Ausführungsformen, detailliert erläutert bzw. in den Ansprüchen definiert.

Eine bevorzugte Möglichkeit, Hybridplasmide mit einer Not I Schnittstelle zu bilden, besteht darin, in die Eco RI Schnittstelle eines Plasmids mit Selektionsmarkern ein Eco RI Fragment mit Not I Schnittstelle, bevorzugt das viertgrößte Eco RI Fragment des Plasmids pBE 3, zu klonieren.

Als Ausgangsplasmid mit Selektionsmarkern wird pBR 322, das keine Not I-Schnittstelle enthält, bevorzugt. Man erhält so pRM Plasmide und in der bevorzugten Ausführungsform das Plasmid pRM 4.

Nach Ligation von mit Not I geschnittenem pBE 3 und pRM Plasmiden, insbesondere pRM 4, und anschließender Transformation kompetenter Wirtszellen, z.B. E. coli, können durch Selektion Zellen gefunden werden, die Hybridplasmide mit verschiedenen Not I-Fragmenten enthalten.

Durch Einsetzen dieser Plasmide in Konjugationssysteme und ihren Transfer von z.B. E. coli nach M. clara, bevorzugt in einem System wie es in der Europäischen Patentanmeldung 0 098 967 beschrieben ist, kann man feststellen, welches der Not I Fragmente die vollständige Information zur Replikation enthält. In jedem Fall ist es das größte Fragment. Die entsprechenden Hybridplasmide werden als pRM-Notori, das bevorzugte Hybridplasmid als pRM4-Notori, bezeichnet.

In der weiteren Beschreibung der Erfindung wird das Vorgehen mit Hilfe des bevorzugt eingesetzten Hybridplasmids pRM4-Notori beschrieben. Analog können jedoch auch andere pRM-Notori Plasmide verwendet werden. Die entsprechenden Bedingungen dazu können in kurzen Vorversuchen ermittelt werden.

Zur weiteren Verkleinerung wird das Plasmid pRM4-Notori mit dem Restriktionsenzym Pvu I behandelt. pRM4-Notori enthält zwei Pvu I Schnittstellen, von denen eine im pBR 322 Anteil des Plasmids liegt, die andere im pBE 3 Anteil, aber außerhalb des für die Replikation notwendigen Not I Fragments. Bei Verdauung des Plasmids pRM4-Notori mit Pvu I entstehen daher zwei Fragmente, von denen das größere alle für eine Replikation in M. clara sowie E. coli notwendigen Funktionen sowie ein funktionelles Tetracyclin-Resistenzgen enthält. Wenn der Restriktionsansatz anschließend mit einer Ligase behandelt wird, so erhält man aus dem größeren der beiden Pvu I Fragmente das Plasmid pSE 1.

Dieses Plasmid kann mit Hilfe eines Konjugationssystems in methylotrophe Bakterien wie Methylomonas clara transferiert werden und verbleibt auch unter nicht-selektiven Bedingungen über viele Generationen stabil in diesem Mikroorganismus.

Mit Hilfe von pSE 1 und eines Plasmids mit Polylinker, beispielsweise pUC 12, kann man die Enden des den Replikationsursprung tragenden Not I Fragments so verändern, daß das den Replikationsursprung tragende Fragment aus pBE 3 nicht mehr durch Not I Schnittstellen, sondern durch eine Kombination anderer Restriktionsschnittstellen begrenzt wird. Auch durch das Anhängen synthetisch hergestellter kurzer DNA-Sequenzen and beide Seiten des den Replikationsursprung tragenden Not I Fragments oder durch eine Verbindung des Not I Fragments mit anderen Polylinker-Sequenzen als die in pUC 12 ist eine solche Veränderung des Not I Fragments möglich. Es entstehen auf diese Art Hybridplasmide, beispielsweise pUC12-Notori, aus denen das den Replikationsursprung tragende Fragment aus pBE 3 durch verschiedene Restriktionsenzyme zugänglich ist, und auf beliebige andere Plasmide übertragen werden kann, die in methylotrophen Bakterien, insbesondere Methylomonas clara, repliziert werden sollen.

Um ein beliebiges Hybridplasmid, das den Replikationsursprung des Plasmids pBE 3 trägt, nach M. clara durch das in der Europäischen Patentanmeldung 0 098 967 beschriebene System zu transferieren, muß auf dem entsprechenden Plasmid eine als "Basis der Mobilisierbarkeit" (bom) bezeichnete DNA-Sequenz vorhanden sein. Diese DNA-Sequenz ist beispielsweise auch auf dem Plasmid pBR 322 zu finden. Ihre Lage ist genau bekannt und kann dem zweitgrößten Hae III-Fragment des Plasmids pBR 322 zugeordnet werden. Dieses Fragment kann analog der oben beschriebenen Vorgehensweise behandelt werden, so daß es durch verschiedene Restriktionszenzyme aus den entsprechenden Hybridplasmiden zugänglich wird.

Mit Hilfe der so konstruierten "origin" bzw. "bom" DNA Sequenz ist es möglich, beliebige Expressionsplasmide so umzuformen, daß sie in methylotrophe Bakterien, insbesondere nach Methylomonas clara, transferiert und dort stabil repliziert werden können. Hierzu wird in einem ersten Schritt das origin-DNA Segment durch eine Kombination geeigneter Restriktionsenzyme, z.B. aus dem Plasmid pUC12-Notori, herausgelöst und mit dem Expressionsvektor verbunden. Sollte auf dem Expressionsvektor die für einen Transfer nach den entsprechenden methylotrophen Bakterien notwendige bom-site nicht vorhanden sein, so wird in einem zweiten Schritt das die bom-site tragende DNA Segment, z.B. aus dem Plasmid pUC12-bom, durch eine Kombination geeigneter Restriktionsenzyme herausgelöst und gleichfalls auf das Plasmid übertragen. Auf diese Weise kann man beliebige Genprodukte codieren, in methylotrophe Bakterien transferieren und durch Fermentation die entsprechenden Genprodukte gewinnen.

Man kann so beispielsweise das Gen für die aromatische Transaminase in Methylomonas clara bringen. Dazu geht man beispielsweise von dem Plasmid pIMS 4004 aus, ein Derivat des Vektors pBR 322, bei dem ein 4,5 kb großes Cla I Fragment aus E.coli D12 Gesamt-DNA in die einzige Cla I Schnittstelle von pBR 322 kloniert worden ist. Dieses 4,5kb große Cla I Fragment trägt das tyrB Gen für die aromatische Transaminase aus E.coli K12. Das Gen erstreckt sich nicht über den gesamten Bereich der 4,5 kb, sondern liegt innerhalb eines durch eine Cla I Schnittstelle sowie eine HindIII Schnittstelle definierten DNA-Abschnitts. Wird das Plasmid pIMS 4004 daher mit Hind III geschnitten, so wird durch die Schnittstelle auf dem 4,5 kb großen Cla I Fragment und der auf dem pBR 322 Anteil vorhandenen Hind III Schnittstelle ein Fragment gebildet, das nach Rezirkularisierung des großen Hind III Fragments deletiert werden kann, ohne das tyrB Gen zu schädigen. Das resultierende Plasmid enthält nur eine Hind III und eine Sal I Schnittstelle, die beide im pBR 322 Anteil des Plasmids liegen. Als Abkömmling von pBR 322 verfügt dieses Plasmid über die für den Transfer in methylotrophe Bakterien notwendige bom-Sequenz, ein funktionelles und gut exprimiertes tyrB Gen, sowie ein zur Selektion zu verwendendes Ampicillin-Resistenzgen. Es sind allerdings keine DNA-Sequenzen vorhanden, die eine Replikation dieses Plasmids in methylotrophen Bakterien erlauben. Um diese Sequenz einzufügen, wird das Plasmid mit den Enzymen Hind III und Sal I vollständig verdaut, ebenso wie das Plasmid pUC12-Notori, wodurch die den Replikationsursprung für methylotrophe Bakterien tragende DNA-Sequenz aus diesem Plasmid herausgelöst wird. Zusätzlich wird das Plasmid pUC12-Notori noch mit dem Restriktionsenzym Pvu I verdaut, um eine Religation des aus pUC12-Notori stammenden Hind III/Sal I Fragments in den Ausgangsvektor zu verhindern. Nach Ligation der gewünschten Gene zu einem Plasmid, wird dieses in das in der Europäischen Patentanmeldung beschriebene Konjugationssystem eingesetzt und in den obligat methylotrophen Bakterienstamm M.clara transferiert. Durch Minilyse der selektionierten Klone und anschließende Restriktionsverdauung der isolierten Plasmide kann sichergestellt werden, daß die zu transferierende DNA in unveränderter Form in den Rezipientenstamm M.clara gelangt ist, in dem sie stabil verbleibt.

Im folgenden wird die Erfindung anhand von Beispielen weitergehend erläutert.

Beispiele

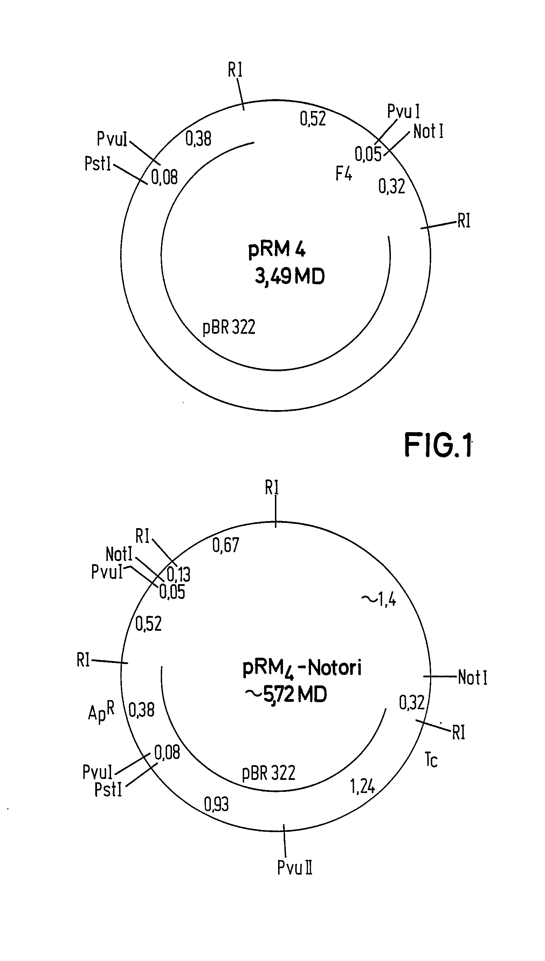

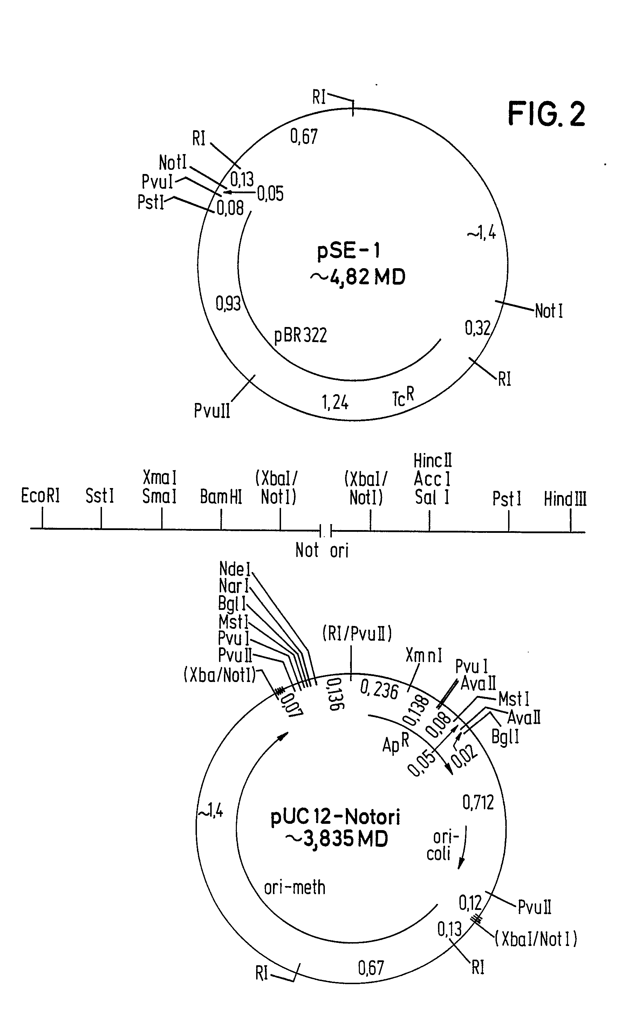

Im Anhang zu den Beispielen sind in der Abbildung 1 die Plasmide pRM 4 und pRM 4-Notori sowie in Abbildung 2 die Plasmide pSE 1 und pUC 12-Notori anhand von Restriktionskarten dargestellt. Es sind nur die für die Erfindung wesentlichen Schnittstellen angegeben. Die Größenangaben sind in MD aufgeführt.

## 1. Konstruktion von pRM4-Nortori und pSE 1

Das Plasmid pRM 4 wurde vollständig mit dem Restriktionsenzym Not I verdaut. Danach wurde die Endonuklease Not I durch Phenolisierung entfernt, die DNA mittels Ethanol gefällt, mit 70%igem Ethanol gewaschen und nach Trocknen im Vakuum in einem geeigneten Volumen an Puffer gelöst, der von der Firma Boehringer Mannheim als optimal für das Enzym alkalische Phosphatase (CIP) angegeben wird. Nach Zugabe von 10 U alkalischer Phosphatase wurde 30 Minuten lang bei 37°C inkubiert, das Enzym durch Phenolisierung aus dem Reaktionsansatz entfernt und die DNA wie oben beschrieben gereinigt. Schließlich wurde sie in TE-Puffer resuspendiert.

Das M. clara Plasmid pBE-3 wurde vollständig mit dem Enzym Not I verdaut, wodurch es in mindestens 9 Fragmente unterschiedlicher Größe aufgespalten wird.

Die Vollständigkeit der Verdauung wurde durch Auftragen eines Aliquots der Inkubationsmischung auf ein Agarosegel überprüft. Anschließend an die Verdauung wurde das Enzym durch Phenolisierung entfernt, die DNA wie vorstehend beschrieben gereinigt und in TE-Puffer resuspendiert.

Das Not I verdaute pRM4 und pBE 3 wurde in verschiedenen Ansätzen in einem molaren Verhältnis von 2:1 über 1:1 bis 1:5 zusammengegeben. Die resultierenden Gemische wurden nach den Angaben des Herstellers (New England Biolabs) mit Puffer versetzt, so daß ein optimales Medium für das Enzym T4-DNA-Ligase resultierte. Die Mischung wurde mit 1 $\mu$l einer Präparation des Enzyms T4-Ligase versetzt (Biolabs, 400U/$\mu$l) und mindestens 14 Stunden lang bei 14 bis 16°C inkubiert. Das Gesamtvolumen des Ansatzes betrug dabei 50 $\mu$l.

Anschließend an die Inkubation wurden 10 $\mu$l des Ligase-Ansatzes abpipettiert und in einem sterilen Eppendorf-Reaktionsgefäß mit 100 $\mu$l einer Suspension von kompetenten Zellen des E. coli Stammes HB101 versetzt. Die Zellen waren nach bekannten Verfahren kompetent für die Aufnahme externer DNA gemacht worden. Es wurde nach dem Verfahren von Cohen et al. transformiert (Proc. Natl. Acad. Sci. 69 (1972) 2110-14), Ampicillin-bzw Tetracyclin-resistente Kolonien wurden auf geeigneten Selektivnährboden isoliert und nach Anzucht in L-Broth (1 % Bacto-Trypton, 0,5 % Hefeextrakt, 0,5 % NaCl) durch Minilyse auf ihren Plasmidgehalt hin untersucht.

Durch Verdauung der Plasmide mit dem Enzym Not I und anschließender Agarose-Gelelektrophorese konnte der Einbau von Not I Fragmenten aus pBE 3 in pRM 4 nachgewiesen werden.

Durch die beschriebene Vorgehensweise konnten die Not I Fragmente 1, 3 und 4 aus pBE 3 in das Plasmid pRM 4 kloniert werden. Die erhaltenen Plasmide wurden in das in der Europäischen Patentanmeldung 0 098 976, Beispiel 10, beschriebene Konjugationssystem eingesetzt und ihr Transfer aus E. coli nach M. clara untersucht. Dabei konnte festgestellt werden, daß nur das Plasmid, welches das Not I Fragment 1 aus pBE 3 in pRM 4 kloniert enthält, zur Ausbildung Tetracyclin-und Ampicillin-resistenter M. clara Klone führte. In diesen Klonen wurde durch Minilyse das entsprechende Plasmid nachgewiesen. Das Plasmid erhielt die Bezeichnung pRM4-Notori.

pRM4-Notori wurde mit dem Enzym Pvu I vollständig geschnitten und der Reaktionsansatz auf 65 °C erhitzt. 10 $\mu$l der Mischung wurden mit Wasser und einem Puffer so verdünnt, daß in einem Gesamtvolumen von 100 $\mu$l das von Hersteller als optimal empfohlene Milieu für das Enzym T4-DNA-Ligase resultierte. Nach Zugabe von 1 $\mu$l T4-DNA-Ligase (Biolabs., 400 U/$\mu$l) wurde 16 Stunden lang bei 16 °C inkubiert. Anschließend wurden kompetente Zellen des E. coli Stammes HB101 mit einem Aliquot des Ligase-Ansatzes transformiert und auf geeigneten Selektionsplatten (L-Broth mit 20 $\mu$g/ml Tetracyclin) Tetracyclin-resistente Kolonien selektioniert. 50 der Tetracyclin-resistenten Klone wurde auf Ampicillin-Platten (L-Broth mit 50 $\mu$g/ml Ampicillin) gepickt. Von 5 Kolonien, die den Phänotyp Tc[r] Ap[s] aufwiesen, wurde durch Minilyse Plasmid-DNA isoliert und untersucht. In allen Fällen bestanden die Plasmide nur noch aus dem großen Pvu I Fragment des Plasmids pRM4-Notori. Diese Hybridplasmide erhielten die Bezeichnung pSE 1.

Das Plasmid pSE 1 wurde in das beschriebene Konjugationssystem eingesetzt und in den plasmidlosen methylotrophen Bakterienstamm M. clara ATCC 31226 transferiert. Die erhaltenen Tetracyclin-resistenten M. clara Klone enthielten alle das erwartete Plasmid pSE 1 in unveränderter Form. Ein Verlust des Plasmids über mehr als 100 Generationen unter nicht-selektiven Bedingungen konnte nicht beobachtet werden.

## 2. Konstruktion von pUC12-Notori

Das Plasmid pSE 1 wurde mit dem Enzym Not I vollständig verdaut und die entstandenen überhängenden Enden durch Behandlung mit dem Enzym DNA-Polymerase I (Klenow-Fragment) in einem von Hersteller, Boehringer Mannheim, empfohlenen Puffer unter den ebenfalls vom Hersteller angegebenen Bedingungen aufgefüllt, so daß glatte Enden resultieren. Nach Abtrennen des Enzyms durch Phenolisierung und Reinigung der DNA mittels Ethanol-Fällung wurde das Fragmentgemisch mit dem Restriktionsenzym Sal I erneut verdaut. Das Enzym Sal I schneidet das Not I Fragment 1 von pBE 3 nicht, wohl aber im Tetracyclin-Resistenzgen des Vektors pSE 1.

Die so vorbehandelte DNA wurde mit pUC 12 DNA, die vollständig mit dem Enzym Xba I verdaut und anschließend mit Klenow-Polymerase aufgefüllt worden war, zusammengegeben und, wie in Beispiel 1 beschrieben, ligiert. Nach Transformation von kompetenten Zellen des E. coli Stammes HB101 wurden Ampicillin-resistente Kolonien selektioniert und nach Anzucht in L-Broth-Medium durch Minilyse auf ihren Plasmidgehalt untersucht. Plasmid-DNA; die gegenüber dem Plasmid pUC 12 einen Größenzuwachs bei der Agarose-Gelelektrophorese aufwies, wurde durch Verdauung mit Eco R I näher charakterisiert. Dadurch konnten Klone identifiziert werden, die das Plasmid pUC 12 mit dem in die aufgefüllte Xba I Schnittstelle klonierten aufgefüllten Not I Fragment 1 des M. clara Plasmids pBE 3 enthielten. Beide möglichen Orientierungen dieses Fragments wurden isoliert. Aus diesen Plasmiden kann der für eine Replikation in M. clara notwendige Bereich des Plasmids pBE 3 in großen Mengen gewonnen und durch Kombination der Restriktionsenzyme Sst I, Sma I, Bam HI und Sal I, Pst I und Hind III isoliert werden.

3. Konstruktion von pUC12-bom

Das handelsübliche Plasmid pUC 12 wurde mit dem Enzym Xba I vollständig verdaut und die gebildeten überhängenden Enden durch Verwendung des Enzyms DNA-Polymerase I (Klenow-Fragment) aufgefüllt. Das Plasmid pBR 322 wurde vollständig mit dem Enzym Hae III verdaut, was zur Ausbildung von 22 Fragmenten mit glatten Enden führt. Die größten dieser Fragmente konnten durch Agarose-Gelelektrophorese voneinander getrennt werden. Die Auftrennung der Fragmente voneinander wurde während der Elektrophorese durch Betrachten des Gels unter UV-Licht in Abständen überprüft, wobei die DNA-Fragmente durch den im Agarosegel enthaltenen Farbstoff Ethidiumbromid durch Fluoreszenz erkennbar wurden.

Als eine ausreichende Trennung der Fragmente voneinander gegeben war, wurde ein Agarose-Scheibchen, welches das zweitgrößte Hae III Fragment von pBR 322 enthielt, mittels eines Skalpells aus dem Gel herausgeschnitten und in einen Dialyseschlauch transferiert. Der Dialyseschlauch wurde mit Puffer [89 mmol/l Tris, 89 mmol/l Borsäure, 2,5 mmol/l EDTA (pH 8,2)] soweit gefüllt, daß das Scheibchen vollständig bedeckt war, in einer Elektrophoresekammer fixiert und in dem gleichen Puffer, der in den Dialyseschlauch gefüllt worden war, eine Elektroelution durchgeführt. Die Elution wurde bei 100 V mindestens 12 Stunden durchgeführt. Dann wurde noch weitere 5 Minuten eluiert, wobei jedoch Plus-und Minuspol gegenüber dem Hauptlauf vertauscht wurden. Der Puffer wurde aus dem Dialyseschlauch in ein Plastikgefäß transferiert. Durch Betrachten des Agarosescheibchens unter UV-Licht wurde sichergestellt, daß alle DNA aus dem Scheibchen eluiert worden war. Der Puffer wurde mindestens zweimal mit Phenol extrahiert, die wäßrigen Phasen vereinigt und mit Ethanol gefällt. Anschließend wurde mindestens dreimal mit 70 %igem Ethanol gewaschen. Nach dem Trocknen im Vakuum wurde die DNA in einem geeigneten Volumen an TE-Puffer aufgenommen und ein Aliquot davon mit pUC 12 DNA, die, wie oben beschrieben, vorbereitet worden war, zusammengegeben und ligiert. Auf L-Broth Platten, die 50 μg/ml Ampicillin enthielten, wurden transformierte E. coli Klone selektioniert und die aus ihnen durch Minilyse isolierte Plasmid DNA durch Agarosegelelektrophorese auf Größenzunahme überprüft.

Es konnten so Klone identifiziert werden, in denen das bom-DNA-Segment aus pBR 322 in beiden Orientierungen kloniert worden war.

**Ansprüche**

1. Hybridplasmid mit einem Fragment aus pBE 3, das für die Replikation in Methylomonas clara verantwortlich ist.

2. DNA-Segment mit dem Replikationsursprung des Plasmids pBE 3, erhältlich durch totale Verdauung von pBE 3 mit Not I und Isolieren des größten Fragments von 2,3 MD.

3. Hybridplasmid nach Anspruch 1, dadurch gekennzeichnet, daß das Fragment aus pBE 3 ein Not I Fragment mit 2,3 MD ist.

4. Hybridplasmid nach Anspruch 1 und 3, dadurch gekennzeichnet, daß ein Plasmid mit Selektionsmarkern darin enthalten ist.

5. Hybridplasmid nach Anspruch 4, dadurch gekennzeichnet, daß pBR 322 darin enthalten ist.

6. Hybridplasmid nach Anspruch 5, dadurch gekennzeichnet, daß ein pRM-Plasmid in diesem Plasmid enthalten ist.

7. Hybridplasmid nach Anspruch 6, dadurch gekennzeichnet, daß pRM 4 in diesem Plasmid enthalten ist.

8. Hybridplasmid nach Anspruch 7, erhältlich durch vollständige Verdauung mit Pvu I und Ringschluß des größten Fragments von 4,82 MD.

9. Verfahren zur Herstellung von Hybridplasmiden mit dem Fragment aus pBE 3 gemäß Anspruch 1 oder 3, dadurch gekennzeichnet, daß man das genannte Plasmid-Fragment von pBE 3 mit einem Plasmid mit Selektionsmarkern ligiert.

10. Verfahren nach Anspruch 9, dadurch gekennzeichnet, daß man das genannte Plasmid-Fragment von pBE 3 mit pRM 4 ligiert.

11. Verfahren nach Anspruch 10, dadurch gekennzeichnet, daß das entstandene Hybridplasmid pRM4-Notori mti Pvu I vollständig geschnitten wird, der Restriktionsansatz mit Ligase behandelt wird und das bei der Verdauung entstandene größte Fragment von 4,82 MD als Plasmid isoliert wird.

12. Die Verwendung des Hybridplasmids mit einem Fragment aus pBE 3, das für die Replikation in Methylomonas clara verantwortlich ist, zur Konstruktion von Expressionsplasmiden für methylotrophe Bakterien.

13. Verwendung des Hybridplasmids nach Anspruch 12 zur Konstruktion von Expressionsplasmiden für Methylomonas clara.

14. Verwendung des Hybridplasmids nach Anspruch 13 zur Konstruktion von Expressionsplasmiden für die aromatische Transaminase in Methylomonas clara.

Patentansprüche für den folgenden Vertragsstaat: Es.

1. Verfahren zur Herstellung von Hybridplasmiden mit einem Fragment aus pBE 3, das für die Replikation in Methylomonas clara verantwortlich ist, dadurch gekennzeichnet, daß man das genannte Plasmid-Fragment von pBE 3 mit einem Plasmid mit Selektionsmarkern ligiert.

2. Verfahren zur Herstellung eines DNA-Segments mit dem Replikationsursprung des Plasmids pBE 3, dadurch gekennzeichnet, daß pBE 3 mit Not I total verdaut wird und das größte Fragment von 2,3 MD isoliert wird.

3. Verfahren nach Anspruch 1, dadurch gekennzeichnet, daß das Fragment aus pBE 3 ein Not I-Fragment mit 2,3 MD ist.

4. Verfahren nach Anspruch 1, dadurch gekennzeichnet, daß man das genannte Plasmid-Fragment von pBE 3 mit pBR 322 ligiert.

5. Verfahren nach Anspruch 1, dadurch gekennzeichnet, daß man das genannte Plasmid-Fragment von pBE 3 mit einem pRM-Plasmid ligiert.

6. Verfahren nach Anspruch 1, dadurch gekennzeichnet, daß man das genannte Plasmid-Fragment von pBE 3 mit pRM 4 ligiert.

7. Verfahren nach Anspruch 6, dadurch gekennzeichnet, daß das entstandene Hybridplasmid pRM 4-Notori mit Pvu I vollständig geschnitten wird, der Restriktionsansatz mit Ligase behandelt wird und das bei der Verdauung entstandene größte Fragment von 4,82 MD als Plasmid isoliert wird.

8. Die Verwendung des Hybridplasmids mit einem Fragment aus pBE 3, das für die Replikation in Methylomonas clara verantwortlich ist, zur Konstruktion von Expressionsplasmiden für methylotrophe Bakterien.

9. Verwendung des Hybridplasmids nach Anspruch 8 zur Konstruktion von Expressionsplasmiden für Methylomonas clara.

10. Verwendung des Hybridplasmids nach Anspruch 9 zur Konstruktion von Expressionsplasmiden für die aromatische Transaminase in Methylomonas clara.

11. Hybridplasmid, erhältlich gemäß Verfahren nach Anspruch 1.

FIG.1

FIG. 2

**pSE-1**
~4,82 MD

pBR 322

RI
0,67
RI
NotI
PvuI
PstI
0,13
0,05
0,08
~1,4
NotI
0,32
RI
0,93
PvuII
1,24
Tc$^R$

EcoRI  SstI  XmaI SmaI  BamHI  (XbaI/NotI)  (XbaI/NotI)  HincII AccI SalI  PstI  HindIII

Not ori

**pUC 12-Notori**
~3,835 MD

ori-meth
ori-coli

NdeI
NarI
BglI
MstI
PvuI
PvuII
(Xba/NotI)
(RI/PvuII)
XmnI
PvuI
AvaII
MstI
AvaII
BglI
0,07
0,136
0,236
0,138
0,08
Ap$^R$
0,05
0,02
0,712
~1,4
0,12
PvuII
0,13
(XbaI/NotI)
RI
RI
0,67